# EUROPEAN PATENT SPECIFICATION

(11) **EP 2 238 108 B1**
(45) Date of publication and mention of the grant of the patent: **29.08.2012**
(21) Application number: 09703697.4
(22) Date of filing: 22.01.2009
(51) Int. Cl.: C07D 209/34, A61K 31/404, A61P 35/00

(54) **SALT FORMS OF A 6-FLUORO-1,2-DIHYDRO-2-OXO-3H-INDOL-3-YLIDENE DERIVATIVE, PROCESS FOR THEIR MANUFACTURE AND PHARMACEUTICAL COMPOSITIONS CONTAINING SAME**
SALZFORMEN EINES 6-FLUOR-1,2-DIHYDRO-2-OXO-3H-INDOL-3-YLIDENDERIVATS, VERFAHREN ZU IHRER HERSTELLUNG UND PHARMAZEUTISCHE ZUSAMMENSETZUNGEN, DIE DIESE ENTHALTEN
FORMES DE SEL D'UN DÉRIVÉ DE 6-FLUORO-1,2-DIHYDRO-2-OXO-3H-INDOL-3-YLIDÈNE, PROCÉDÉ DE FABRICATION ET COMPOSITIONS PHARMACEUTIQUES LE CONTENANT

(30) Priority: 25.01.2008 EP 08150660
(43) Date of publication of application: 13.10.2010
(73) Proprietor: Boehringer Ingelheim International GmbH, 55216 Ingelheim (DE)
(72) Inventor: WERTHMANN, Ulrike, 55216 Ingelheim Am Rhein (DE); KULINNA, Christian, 55216 Ingelheim Am Rhein (DE); PFRENGLE, Waldemar, 55216 Ingelheim Am Rhein (DE); RALL, Werner, 55216 Ingelheim Am Rhein (DE)
(74) Representative: Hammann, Heinz
(86) International application number: PCT/EP2009/000378
(87) International publication number: WO 2009/092581

(56) References cited:
- US-A1- 2005 043 389

## Description

The present invention relates to a salt form of a 6-fluoro-1,2-dihydro-2-oxo-3H-indol-3-ylidene derivative, namely the Monohydrochloride salt of the 4-[(Z)-[[4-[(dimethylamino)methyl]phenyl]amino](6-fluoro-1,2-dihydro-2-oxo-3H-indol-3-ylidene)methyl]-benzenepropanoic acid, to a process for its manufacture and to pharmaceutical compositions containing this salt.

The compound 4-[(Z)-[[4-[(dimethylamino)methyl]phenyl]amino](6-fluoro-1,2-dihydro-2-oxo-3H-indol-3-ylidene)methyl]-benzenepropanoic acid is depicted below as Formula I.

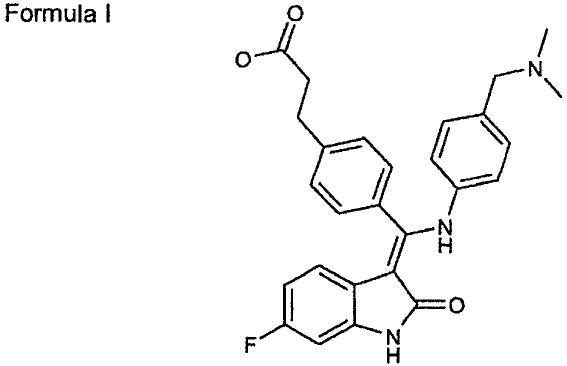

### Background to the invention

A number of 2-indolinone derivatives are already known in the prior art. Thus, for example, International Patent Applications WO 01/27081, WO 04/009546 and WO 04/009547 disclose 2-indolinone derivatives which have valuable pharmacological properties.

The compound of above formula I is disclosed in WO 04/009546 and WO 04/009547. In WO 04/009547, it is disclosed as example 10.1. A process for the manufacturing of this compound is disclosed in this patent application as well. This manufacturing process is described in WO 04/009547, under Example 10.1 and using the starting material of Example VI.22.

Like the 2-indolinone derivatives mentioned in the prior art, the compound of above Formula I also has, in particular, an inhibiting effect on various kinases, particularly receptor tyrosine kinases such as VEGFR1, VEGFR2, VEGFR3, PDGFRα, PDGFRβ, FGFR1, FGFR3, EGFR, HER2, c-Kit, IGF1R, Flt-3 and HGFR, and on the proliferation of cultivated human cells, particularly endothelial cells, e.g. in angiogenesis, but also on the proliferation of other cells, particularly tumour cells.

The pharmacologically valuable properties of the indolinone derivatives disclosed in the prior art and mentioned above constitute the basic prerequisite for an effective use of these compounds in pharmaceutical compositions. An active substance must in any case satisfy additional requirements in order to be accepted for use as a drug. These parameters are largely connected with the physicochemical nature of the active substance.

Without being restrictive, examples of these parameters are the preservation of activity of the active substance under various environmental conditions, the stability during production of the pharmaceutical formulation and stability in the final compositions of the active substance or of the drug. The pharmaceutically active substance used to prepare the pharmaceutical compositions should therefore have great stability which is ensured even under all kinds of environmental conditions. This is absolutely essential to prevent pharmaceutical compositions being used which contain breakdown products, for example, in addition to the active substance itself. In such a case the content of active substance present in the pharmaceutical formulation might be lower than specified.

The absorption of moisture reduces the content of pharmaceutically active substance as a result of the increased weight caused by the uptake of water. Pharmaceutical compositions with a tendency to absorb moisture have to be protected from moisture during storage, e.g. by the addition of suitable drying agents or by storing the drug in an environment where it is protected from moisture. In addition, the uptake of moisture may reduce the content of pharmaceutically active substance during manufacture if the pharmaceutical substance is exposed to the environment without being protected from moisture in any way. Preferably, therefore, a pharmaceutically active substance should be only slightly hygroscopic.

As the crystal stability of an active substance is an important factor for maintaining the content of active substance in a preparation stable, there is a need to clarify as far as possible any existing polymorphism of an active substance present in crystalline form. If polymorphic modifications of an active substance occur under certain conditions, care must be taken to ensure that the crystalline modification of the substance does not change in the pharmaceutical preparation later produced from it. Otherwise, this could have a harmful effect on the reproducible potency of the drug. Generally, active substances characterised by only slight polymorphism are thus preferred.

The problem underlying the present invention is thus the provision of a pharmaceutically active substance in an improved form which is not only characterised by high pharmacological potency but also satisfies the abovementioned physicochemical requirements.

### Summary of the invention

This problem is solved by the specific salt form of the compound 4-[(Z)-[[4-[(dimethylamino)methyl]phenyl]amino](6-fluoro-1,2-dihydro-2-oxo-3H-indol-3-ylidene)methyl]-benzenepropanoic acid in accordance with the present invention. This specific salt form is the chloride salt form.

A first object of the present invention is thus the monohydrochloride hemihydrate crystalline salt form of the compound 4-[(Z)-[[4-[(dimethylamino)methyl]phenyl]amino](6-fluoro-1,2-dihydro-2-oxo-3H-indol-3-ylidene)methyl]-benzenepropanoic acid.

A further object of the present invention is a process for the manufacture of the above mentioned salt form of the compound 4-[(Z)-[[4-[(dimethylamino)methyl]phenyl]amino](6-fluoro-1,2-dihydro-2-oxo-3H-indol-3-ylidene)methyl]-benzenepropanoic acid, process which is described hereafter in the experimental section.

A further object of the present invention is a pharmaceutical composition containing the monohydrochloride hemihydrate salt form of the compound 4-[(Z)-[[4-[(dimethylamino)methyl]phenyl]amino](6-fluoro-1,2-dihydro-2-oxo-3H-indol-3-ylidene)methyl]-benzenepropanoic acid.

### Brief description of the Figures

Figure 1 shows the thermoanalysis and determination of the melting point (DSC), and the determination of the weight loss (TG) of crystalline 4-[(Z)-[[4 [(dimethylamino)methyl]phenyl]amino](6-fluoro-1,2-dihydro-2-oxo-3H-indol-3-ylidene)methyl]-benzenepropanoic acid, monohydrochloride.
Figure 2 shows the X-ray powder diffractogram of crystalline 4-[(Z)-[[4-[(dimethylamino)methyl]phenyl]amino](6-fluoro-1,2-dihydro-2-oxo-3H-indol-3-ylidene)methyl]-benzenepropanoic acid, monohydrochloride.

### Detailed description of the invention

The compound 4-[(Z)-[[4-[(dimethylamino)methyl]phenyl]amino](6-fluoro-1,2-dihydro-2-oxo-3H-indol-3-ylidene)methyl]-benzenepropanoic acid is described in WO 04/009547, however using a different nomenclature, namely 3-Z-[1-(4-dimethylaminomethylanilino)-1-(4-(2-carboxyethyl)phenyl)methylene]-6-fluoro-2-indolinone. A process for the manufacture of this compound is also described in WO 04/009547, the content of which is incorporated herein by reference.

The manufacturing processes to obtain the compound 4-[(Z)-[[4-[(dimethylamino)methyl]phenyl]amino](6-fluoro-1,2-dihydro-2-oxo-3H-indol-3-ylidene)methyl]-benzenepropanoic acid and the salts of this compound in accordance with the present invention are described in the following.

These processes are illustrative of the present invention and shall not represent a limitation of the scope of the present invention.

### Process for the synthesis of the compound 4-[(Z)-[[4-[(dimethylamino)methyl]phenyl]amino](6-fluoro-1,2-dihydro-2-oxo-3H-indol-3-ylidene)methyl]-benzenepropanoic acid

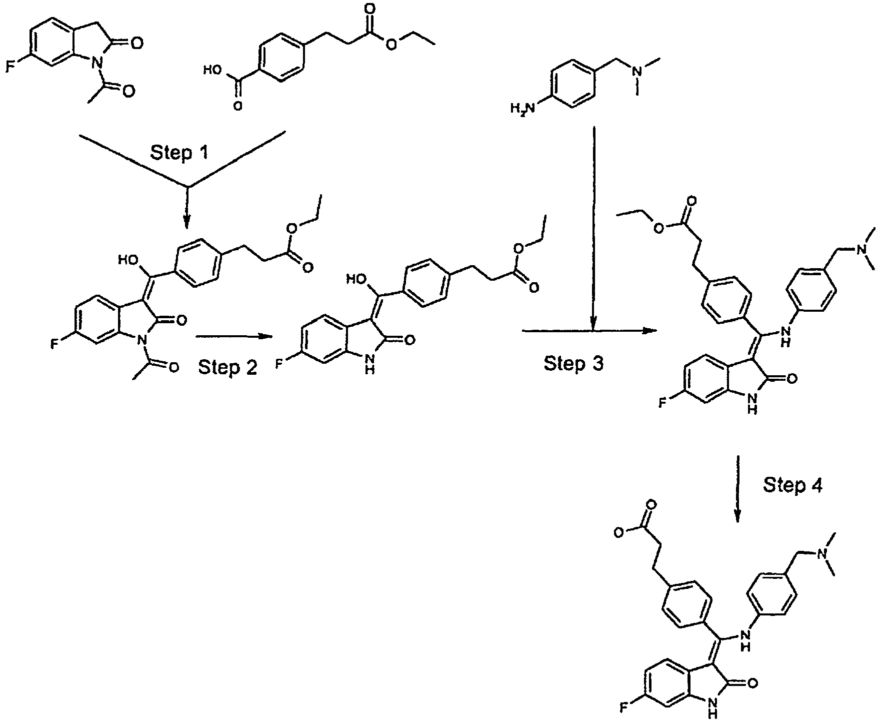

In the following, the experimental details of the synthesis are described.

The starting compounds and reagents are all commercially available.
6-fluoro-oxindole (6-fluoro-2-indolinone), CAS 56341-39-0, is commercially available.
2,5-difluoronitrobenzene, CAS 364-74-9, for the synthetic route described in WO 04/009547 in Examples I - IV, is commercially available.
4-carboxybenzaldehyde, CAS 619-66-9, used for the synthesis of 4-(2-ethoxycarbonylethyl)benzoic acid (preparation analogously to Tetrahedron 1997, 53, 7335-7340), is commercially available.
4-amino-N,N-dimethyl-benzenemethanamine, CAS 6406-74-2, is commercially available.

### Synthesis step 1

### Synthesis of 4-[(E)-(1-acetyl- 6-fluoro-1,2-dihydro-2- oxo-3H-indol-3-ylidene) hydroxymethyl]-benzenepropanoic acid, ethyl ester

This synthesis step is described in WO 04/009547, under Example 10.1 and using the starting material of Example VI.22.
4-[(E)-(1-acetyl- 6-fluoro-1,2-dihydro-2- oxo-3H-indol-3-ylidene) hydroxymethyl]-benzenepropanoic acid, ethyl ester, or 1-acetyl-3-[1-hydroxy-1-(4-(2-ethoxycarbonylethyl)phenyl)methylene]-6-fluoro-2-indolinone, is prepared from 1-acetyl-6-fluoro-2-indolinone (described in WO 04/009547, under Example V) and 4-(2-ethoxycarbonylethyl)benzoic acid (preparation analogously to Tetrahedron 1997, 53, 7335-7340).

### Synthesis step 2

### Synthesis of 4-[(E)-(6-fluoro-1,2-dihydro-2- oxo-3H-indol-3-ylidene) hydroxymethyl]-benzenepropanoic acid, ethyl ester

1.62kg (4.077 mol) 4-[(E)-(1-acetyl-6-fluoro-1,2-dihydro-2-oxo-3H-indol-3-ylidene)hydroxymethyl]-benzenepropanoic acid, ethyl ester are suspended in 14 L methanol, and 220g (3.873mol) sodium-methoxide are added. After stirring for 1 hour under reflux the solution is cooled to 15°C. 340ml (4.079mol) hydrochloride acid 37% in 3.7 L water is added at 15°C. The obtained precipitate is suction filtered, washed with 8 litres of water/methanol in proportion 1:1 and dried at 60°C.

| | |
|---|---|
| Yield: | 1.29 kg (89% of theory) |
| T_{m.p.} = | 163°C (DSC 10K/min) |
| Purity according to HPLC: | 95.2% (column: Prontosil 120-3-C18, 3 µm) |
| Empirical formula: | C₂₀H₁₈FNO₄ |
| ESI mass spectrum: | m/z = 356 [M+H]⁺ |

### Synthesis step 3

### Synthesis of 4-[(Z)-[[4-[(dimethylamino)methyl]phenyl]amino](6-fluoro-1,2-dihydro-2-oxo-3H-indol-3-ylidene)methyl]-benzenepropanoic acid, ethyl ester

3.07kg (4.444 mol) 4-[(E)-(6-fluoro-1,2-dihydro-2-oxo-3H-indol-3-ylidene)hydroxymethyl]-benzenepropanoic acid, ethyl ester are suspended in 7.0 L dioxane. After addition of 1100ml (8.639mol) trimethylsilylchloride and 1.363kg (9.071) 4-amino-N,N-dimethyl-benzenemethanamine, the temperature is raised up to about 30°C. 3.65 L (17.278mol) hexamethyldisilazane and 4.2 L dioxane are added. The mixture is heated to about 100°C and stirred for about 60 hours. After cooling to about 60°C and carefully addition of 12 L ethanol the solvents are evaporated under vacuum. The residue is dissolved in 10 L ethanol under reflux. The solution is cooled to about 8°C and the obtained precipitate is suction filtered, washed with 3.2 litres of ethanol and dried at 45°C under vacuum.

| | |
|---|---|
| Yield: | 3.355 kg (79.7% of theory) |
| T_{m.p}. = | 159 °C (DSC 10K/min) |
| Purity according to HPLC: | 99.1 % (column: Prontosil 120-3-C18, 3 µm) |
| Empirical formula: | C₂₉H₃₀ FN₃O₃ |
| ESI mass spectrum: | m/z = 488 [M+H]⁺ |

### Synthesis step 4

### Synthesis of 4-[(Z)-[[4-[(dimethylamino)methyl]phenyl]amino](6-fluoro-1,2-dihydro-2-oxo-3H-indol-3-ylidene)methyl]-benzenepropanoic acid,

1055 g (2.164 mol) of 4-[(Z)-[[4-[(dimethylamino)methyl]phenyl]amino](6-fluoro-1,2-dihydro-2-oxo-3H-indol-3-ylidene)methyl]-benzenepropanoic acid, ethyl ester are suspended in 8.9 L of methanol. 4330 ml of 1 mol/l sodium hydroxide solution are added and the mixture is heated to about 70°C. After stirring for another two hours at about 70°C the solution is cooled to about 20°C. 2200ml of 1 mol/l hydrochloride acid is added, the yellow precipitate formed is suction filtered and washed with water. The substance is dried under vacuum at 55°C.

| | |
|---|---|
| Yield: | 939 g (94.4% of theory), |
| T_{m.p.}= | 176°C |
| Empirical formula: | C₂₇H₂₆ FN₃O₃ |
| ESI mass spectrum: | m/z = 460 [M+H]⁺ |
| Water content: | 2.5% (KF) direct after drying |
| | 6-10% (KF) after equlibration on air |

### Process for the synthesis of the monohydrochloride salt form of 4-[(Z)-[[4-[(dimethylamino)methyl]phenyl]amino](6-fluoro-1,2-dihydro-2-oxo-3H-indol-3-ylidene)methyl]-benzenepropanoic acid

927 g (1.941 mol) of 4-[(Z)-[[4-[(dimethylamino)methyl]phenyl]amino](6-fluoro-1,2-dihydro-2-oxo-3H-indol-3-ylidene)methyl]-benzenepropanoic acid are suspended in 9 litres of acetone. 2020 ml (2.02 mol) of hydrochloride acid (1mol/L) are added. After one minute crystallisation sets in. 15 L ethylacetate are added and the obtained suspension is cooled to about -3°C. The yellow precipitate is suction filtered, washed with 2.5 litres of ethylacetatelacetone in proportion 1:1 and dried at 55°C under vacuum.

| | |
|---|---|
| Yield: | 851 g (88.4% of theory) |
| T_{m.p}. = | 282°C (DSC 10K/min) |
| Purity according to HPLC: | 99.64% (column: Prontosil 120-3-C18, 3 µm) |
| Empirical formula: | C₂₇H₂₆ FN₃O₃ x HCL |
| ESI mass spectrum: | m/z = 460 [M+H]⁺ |
| Water content: | 2.1%(KF) |

In the foregoing, the characteristics and properties of the salt form in accordance with the present invention are discussed.

The monohydrochloride salt form of the compound according to the invention, namely 4-[(Z)-[[4-[(dimethylamino)methyl]phenyl]amino](6-fluoro-1,2-dihydro-2-oxo-3H-indol-3-ylidene)methyl]-benzenepropanoic acid monohydrochloride, is a yellow crystalline powder with irregular shaped crystals. No significant change in crystallinity is observed after intensive milling or compression. In addition it is slightly hygroscopic, but no change in crystal modification is observed.

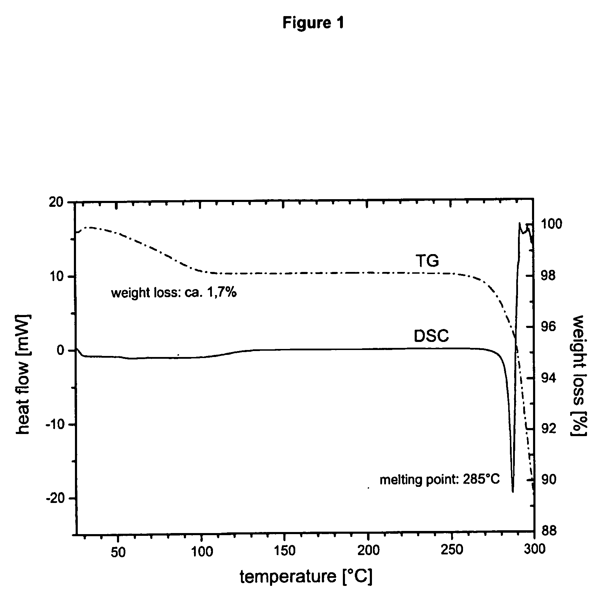

The crystalline form of the monohydrochloride salt according to the invention is characterised by a melting point of 285°C ± 5°, as determined by Differential Scanning Calorimetry (DSC; Peak maximum evaluation using a DSC 821 made by Mettler Toledo and a heating rate of 10°C/min). The DSC diagram is depicted in Figure 1.

The crystalline form of the monohydrochloride salt according to the invention is further characterised by a weight loss of ca. 1,6-1,8% water up to 130°C, as determined by Thermal Gravimetry (TG). Under standard conditions, the monohydrochloride salt form of the compound according to the invention is present in the form of the hemihydrate, from which water escapes at a temperature of about 130°C. The TG diagram is also depicted in Figure1.

Thus, the monohydrochloride salt form of the compound in accordance with the present invention is a crystalline salt form characterised by a melting point of T_{m.p.} = 285 ± 5°C (Determined by DSC; Evaluation by peak maximum; Heating rate: 10°C/min) and a weight loss of ca. 1,6- 1,8% water up to 130°C (Determined by TG).

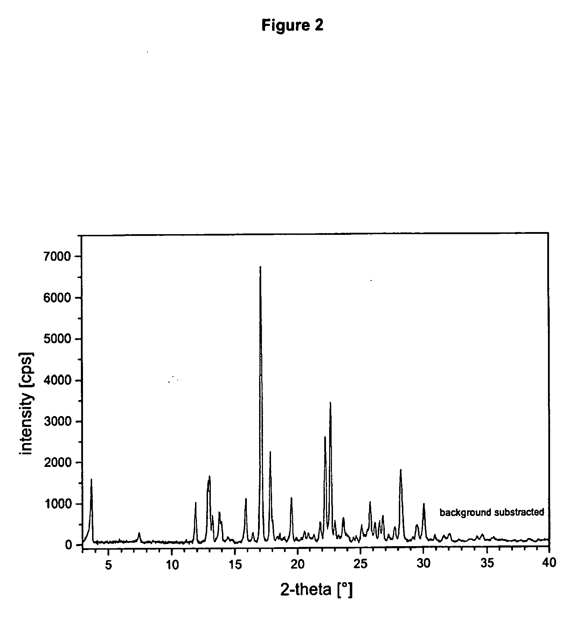

The crystalline form of the monohydrochloride salt according to the invention can be further characterised by specific X-ray powder diffraction values. The X-ray powder diffractogram of the crystalline form recorded using a STOE - STADI P-diffractometer in transmission mode fitted with a location-sensitive detector (OED) and a Cu-anode as X-ray source (CuK_{α} radiation, λ = 1,54056 Å, 40kV, 40mA), is depicted in Figure 2. The following Table I contains the related diffraction values.

**Table I**

| **2** Θ **[°]** | **d-value [Å]** | **rel. Int. [%]** |
|---|---|---|
| 3.71 | 23.82 | 24 |
| 7.41 | 11.91 | 4 |
| 11.89 | 7.44 | 14 |
| 12.90 | 6.86 | 22 |
| 13.00 | 6.81 | 25 |
| 13.24 | 6.68 | 11 |
| 13.82 | 6.40 | 11 |
| 13.97 | 6.33 | 7 |
| 14.45 | 6.12 | 3 |
| 15.89 | 5.57 | 16 |
| 16.45 | 5.38 | 4 |
| 17.12 | 5.17 | 100 |
| 17.85 | 4.97 | 33 |
| 18.02 | 4.92 | 9 |
| 18.38 | 4.82 | 2 |
| 18.59 | 4.77 | 4 |
| 18.90 | 4.69 | 2 |
| 19.54 | 4.54 | 17 |
| 19.90 | 4.46 | 2 |
| 20.33 | 4.37 | 2 |
| 20.57 | 4.31 | 5 |
| 20.88 | 4.25 | 4 |
| 21.33 | 4.16 | 3 |
| 21.83 | 4.07 | 8 |
| 22.26 | 4.00 | 38 |
| 22.68 | 3.92 | 51 |
| 23.01 | 3.86 | 8 |
| 23.29 | 3.82 | 3 |
| 23.68 | 3.75 | 9 |
| 24.44 | 3.64 | 2 |
| 24.66 | 3.61 | 3 |
| 25.12 | 3.54 | 6 |
| 25.78 | 3.45 | 14 |
| 26.17 | 3.40 | 8 |
| 26.52 | 3.36 | 8 |
| 26.80 | 3.32 | 10 |
| 27.23 | 3.27 | 3 |
| 27.75 | 3.21 | 6 |
| 28.22 | 3.16 | 26 |
| 29.16 | 3.06 | 2 |
| 29.51 | 3.02 | 6 |
| 30.04 | 2.97 | 14 |

In the above Table I the value "2 Θ [°]" denotes the angle of diffraction in degrees and the value "dₕₖₗ [Å]" denotes the specified distances in A between the lattice planes.

According to the values shown in the above Table I, the crystalline form of the monohydrochloride salt in accordance with the present invention is crystalline 4-[(Z)-[[4-[(dimethylamino)methyl]phenyl]amino](6-fluoro-1,2-dihydro-2-oxo-3H-indol-3-ylidene)methyl]-benzenepropanoic acid monohydrochloride, characterised by X-ray powder diagram values of d = 6.81 Å, 5.17 Å, 4.92 Å, 4.00 Å, 3.92 Å and 3.16 Å having an intensity of more than 25%.

As can be seen from the above results, the monohydrochloride salt form of the compound in accordance with the present invention has unexpectedly good properties for pharmaceutical development and good stability properties, especially a high and sharp melting point, a slight hygroscopicity and a stable crystallinity in the form of an hemihydrate.

Other salts forms namely the maleinate, fumarate, citrate, succinate, tartrate, bromide, sulfate or sodium salt forms may as well be suitable for pharmaceutical development, these have less good properties, especially with respect to their crystalline stability and their dissolution.

For example, the sodium and tartrate salt forms have less good crystallinity properties, which affects the stability of the drug substance. As can be seen by differential scanning calorimetry (DSC), the sodium crystalline salt form shows a dehydration/melting in a broad endothermal reaction up to 110°C and an exothermal recrystallization reaction at 212°C. The tartrate crystalline salt form shows three endothermal reactions at 65°C, 138°C and 167°C and two sharp endothermal reactions at 221°C and 232 °C. In comparison, the monohydrochloride crystalline salt form shows a sharp melting point at 285°C, the succinate crystalline salt form shows a sharp melting point at 242°C and the bromide crystalline salt form shows a sharp melting point at 279°C. The monohydrochloride, the succinate and the bromide crystalline salt forms further show a loss of weight of, respectively, ca. 1.7%, ca. 1,5% and ca. 2,2% as measured by thermal gravimetry (TG), which is characteristic of a slight hygroscopicity, whereas the sodium and tartrate salt forms show a loss of weight of, respectively, ca. 15% and ca. 4,3% as measured by thermal gravimetry (TG), which is characteristic of a strong hygroscopicity. Thus, the chloride, succinate and bromide salt forms have unexpectedly better crystalline properties for pharmaceutical development than the sodium and tartrate salt forms.

Furthermore, although all the above mentioned salt forms are characterized by a low solubility over almost the whole pH range, the chloride salt form presents the advantage of a better dissolution rate. Hence, common to the chloride, succinate and bromide salt form is a rather low solubility in aqueous media over a wide pH-range. The succinate and bromide salt forms show in addition also a very slow intrinsic dissolution rate, only the chloride form exhibits an acceptable fast intrinsic dissolution rate.

Furthermore, the compound in accordance with the present invention may be used on its own or in conjunction with other pharmacologically active substances.

Suitable preparations for the pharmaceutical compositions in accordance with the present invention include for example tablets, capsules, suppositories, solutions, elixirs, emulsions or dispersible powders. The proportion of the pharmaceutically active compound(s) should be in the range from 0.01 to 90 wt.-%, preferably 0.1 to 50 wt.-% of the composition as a whole, i.e. in amounts which are sufficient to achieve the dosage necessary to achieve a therapeutic effect. If necessary the doses specified may be given several times a day.

Suitable tablets may be obtained, for example, by mixing the active substance(s) with known excipients, for example inert diluents such as calcium carbonate, calcium phosphate or lactose, disintegrants such as maize starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number or layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavour enhancer, e.g. a flavouring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of isotonic agents, preservatives such as p-hydroxybenzoates, or stabilisers such as alkali metal salts of ethylenediamine tetraacetic acid, optionally using emulsifiers and/or dispersants, whilst if water is used as the diluent, for example, organic solvents may optionally be used as solvating agents or dissolving aids, and transferred into injection vials or ampoules or infusion bottles.

Capsules containing one or more active substances or combinations of active substances may for example be prepared by mixing the active substances with inert carriers such as lactose or sorbitol and packing them into gelatine capsules. An especially suitable pharmaceutical formulation for the compounds in accordance with the present invention is soft gelatine capsules. Suitable soft gelatine capsules for the encapsulation of pharmaceutical compounds and the process for their preparation are described, for example, in GB patent No. 395546, US patent No. 2,720,463, US patent No. 2,870,062, US patent No. 4,829,057, and in the following publications: ANON (Verpack-Rundsch., Vol. 21, No. 1 Jan 1970, pp, 136-138), Lachman et al. (The Theory and Practice of Industrial Pharmacy, Chap. 13, published by Lea & Febiger, 1970), Ebert (Soft Gelatine Capsules: A Unique Dosage Form, reprint from Pharmaceutical Technology, Oct. 1977) and R. F. Jimerson (Soft Gelatine Capsule Update, Drug Development and Industrial Pharmacy, Vol. 12 (8 & 9), pp. 1133-1144, 1986).

Suitable suppositories may be made for example by mixing with carriers provided for this purpose, such as neutral fats or polyethyleneglycol or the derivatives thereof.

Excipients which may be used include, for example, water, pharmaceutically acceptable organic solvents such as paraffins (e.g. petroleum fractions), vegetable oils (e.g. groundnut or sesame oil), mono- or polyfunctional alcohols (e.g. ethanol or glycerol), carriers such as e.g. natural mineral powders (e.g. kaolins, days, talc, chalk), synthetic mineral powders (e.g. highly dispersed silicic acid and silicates), sugars (e.g. cane sugar, lactose and glucose) emulsifiers (e.g. lignin, spent sulphite liquors, methylcellulose, starch and polyvinylpyrrolidone) and lubricants (e.g. magnesium stearate, talc, stearic acid and sodium lauryl sulphate).

The preparations are administered by the usual methods, preferably by oral route, by injection or transdermally. For oral administration the tablets may of course contain, apart from the abovementioned carriers, additives such as sodium citrate, calcium carbonate and dicalcium phosphate together with various additives such as starch, preferably potato starch, gelatine and the like. Moreover, lubricants such as magnesium stearate, sodium lauryl sulphate and talc may be used at the same time for the tabletting process. In the case of aqueous suspensions the active substances may be combined with various flavour enhancers or colourings in addition to the excipients mentioned above. For parenteral use, solutions of the active substances with suitable liquid carriers may be used.

The dosage for intravenous use is from 1 - 1000 mg per hour, preferably between 5 and 500 mg per hour.

However, it may sometimes be necessary to depart from the amounts specified, depending on the body weight, the route of administration, the individual response to the drug, the nature of its formulation and the time or interval over which the drug is administered. Thus, in some cases it may be sufficient to use less than the minimum dose given above, whereas in other cases the upper limit may have to be exceeded. When administering large amounts it may be advisable to divide them up into a number of smaller doses spread over the day.

The following examples of formulations illustrate the present invention without restricting its scope.

| A) | Tablets | per tablet |
|---|---|---|
| | active substance | 100 mg |
| | lactose | 140 mg |
| | maize starch | 240 mg |
| | polyvinylpyrrolidone | 15 mg |
| | magnesium stearate | 5 mg |
| | | 500 mg |

The finely ground active substance, lactose and some of the maize starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| B) | Tablets | per tablet |
|---|---|---|
| | active substance | 80 mg |
| | lactose | 55 mg |
| | maize starch | 190 mg |
| | microcrystalline cellulose | 35 mg |
| | polyvinylpyrrolidone | 15 mg |
| | sodium-carboxymethyl starch | 23 mg |
| | magnesium stearate | 2 mg |
| | | 400 mg |

The finely ground active substance, some of the maize starch, lactose, microcrystalline cellulose and polyvinylpyrrolidone are mixed together, the mixture is screened and worked with the remaining maize starch and water to form a granulate which is dried and screened. The sodium carboxymethyl starch and the magnesium stearate are added and mixed in and the mixture is compressed to form tablets of a suitable size.

## Claims

1. 4-[(Z)-[[4-[(dimethylamino)methyl]phenyl]amino](6-fluoro-1,2-dihydro-2-oxo-3H-indol-3-ylidene)methyl]-benzenepropanoic acid, monohydrochloride hemihydrate in crystalline form.

2. 4-[(Z)-[[4-[(dimethylamino)methyl]phenyl]amino](6-fluoro-1,2-dihydro-2-oxo-3H-indol-3-ylidene)methyl]-benzenepropanoic acid, monohydrochloride hemihydrate in crystalline form in accordance with claim 1, **characterised by** a melting point of T_{m.p.} = 285 ± 5°C (determined by Differential Scanning Calorimetry; evaluation using peak-maximum; heating rate: 10°C/min).

3. 4-[(Z)-[[4-[(dimethylamino)methyl]phenyl]amino](6-fluoro-1,2-dihydro-2-oxo-3H-indol-3-ylidene)methyl]-benzenepropanoic acid, monohydrochloride hemihydrate in crystalline form in accordance with claim 1, **characterised by** an X-Ray Powder Diagram showing the characteristic values d = 6.81 Å, 5.17 Å, 4.92 Å, 4.00 Å, 3.92 Å and 3.16 Å with an intensity of more than 25% (recorded using a STOE - STADI P-diffractometer in transmission mode fitted with a location-sensitive detector .(OED) and a Cu-anode as X-ray source (CuK_{α} radiation, λ = 1,54056 Å, 40kV, 40mA)) .

4. Pharmaceutical composition containing the 4-[(Z)-[[4-[(dimethylamino)methyl]phenyl]amino](6-fluoro-1,2-dihydro-2-oxo-3H-indol-3-ylidene)methyl]-benzenepropanoic acid, monohydrochloride hemihydrate in crystalline form and one or more inert carriers and/or diluents.

## Patentansprüche

1. 4-[(Z)-[[4-[(Dimethylamino)methyl]phenyl]amino (6-fluor-1,2-dihydro-2-oxo-3H-indol-3-yliden)methyl]-benzolpropansäure-Monohydrochloridhemihydrat in kristalliner Form.

2. 4-[(Z)-[[4-[(Dimethylamino)methyl]phenyl]amino](6-fluor-1,2-dihydro-2-oxo-3H-indol-3-yliden)methyl]-benzolpropansäure-Monohydrochloridhemihydrat in kristalliner Form nach Anspruch 1, **gekennzeichnet durch** einen Schmelzpunkt T_{Schmp}. = 285 ± 5°C (bestimmt **durch** Differentialscanningkalorimetrie; Auswertung unter Verwendung des Peakmaximums; Aufheizrate: 10°C/min.).

3. 4-[(Z)-[[4-[(Dimethylamino)methyl]phenyl]amino](6-fluor-1,2-dihydro-2-oxo-3H-indol-3-yliden)methyl]-benzolpropansäure-Monohydrochloridhemihydrat in kristalliner Form nach Anspruch 1, **gekennzeichnet durch** ein Röntgenpulverdiffraktogramm, das die charakterisitischen Werte d = 6,81Å, 5,17Å, 4,92Å, 4,00Å, 3,92Å und 3,16Å mit einer Intensität von mehr als 25% zeigt (aufgenommen unter Verwendung eines STOE STADI P-Diffraktometers im Transmissionsmodus, ausgerüstet mit einem ortssensitiven Detektor (OED) und einer Cu-Anode als Röntgenquelle (CuK_{α}-Strahlung, λ=1,54056Å, 40 kV, 40 mA)).

4. Pharmazeutische Zusammensetzung, enthaltend 4-[(Z)-[[4-[(Dimethylamino)methyl]-phenyl] amino] (6-fluor-1,2-dihydro-2-oxo-3H-indol-3-yliden)methyl]-benzolpropansäure-Monohydrochloridhemihydrat in kristalliner Form und ein oder mehr inerte Träger und/oder Verdünnungsmittel.

## Revendications

1. Monohydrochlorure semi-hydraté de l'acide 4-[(Z)-[[4-[(diméthylamino)méthyl]phényl]amino](6-fluoro-1,2-dihydro-2-oxo-3H-indol-3-ylidène)-méthyl]benzène-propanoïque sous forme cristalline.

2. Monohydrochlorure semi-hydraté de l'acide 4-[(Z)-[[4-[(diméthylamino)méthyl]phényl]amino] (6-fluoro-1,2-dihydro-2-oxo-3H-indol-3-ylidène)-méthyl]-benzène-propanoïque sous forme cristalline selon la revendication 1, **caractérisé par** un point de fusion T_{f} = 285 ± 5°C (déterminé par analyse calorimétrique différentielle à compensation de puissance ; évaluation au moyen du maximum du pic ; vitesse de chauffage : 10°C/minute).

3. Monohydrochlorure semi-hydraté de l'acide 4-[(Z)-[[4-[(diméthylamino)méthyl]phényl]amino](6-fluoro-1,2-dihydro-2-oxo-3H-indol-3-ylidène)méthyl]benzène-propanoïque sous forme cristalline selon la revendication 1, **caractérisé par** un spectre de diffraction des rayons X sur poudre présentant les valeurs caractéristiques d = 6,81 Å, 5, 17 Å, 4,92 Å, 4,00 Å, 3,92 Å et 3,16 Å avec une intensité supérieure à 25 % (enregistré au moyen d'un diffractomètre STOE STADI-P en mode transmission équipé d'un détecteur sensible à la position (OED) et d'une anode en Cu en tant que source de rayons X (rayonnement CuK_{α}, λ = 1,54056 Å, 40 kV, 40 mA).

4. Composition pharmaceutique contenant le monohydrochlorure semi-hydraté de l'acide 4-[(Z)-[[4-[(diméthylamino)méthyl]phényl]amino](6-fluoro-1,2-dihydro-2-oxo-3H-indol-3-ylidène)-méthyl]-benzène-propanoïque sous forme cristalline et un ou plusieurs supports et/ou diluants inertes.
